# EUROPEAN PATENT APPLICATION

(11) **EP 4 796 559 A1**
(43) Date of publication of application: **26.08.2026**
(21) Application number: 24878469.6
(22) Date of filing: 21.05.2024
(51) Int. Cl.: C07K 14/47, C12N 15/12, A61K 38/17

(54) **KERATIN CF6, PREPARATION METHOD THEREFOR, AND PHARMACEUTICAL COMPOSITION AND USE THEREOF**

(30) Priority: 18.10.2023 CN 202311346872
(71) Applicant: Institute of Materia Medica, Chinese Academy of Medical Sciences, Beijing 100050 (CN)
(72) Inventor: YU, Shishan, Beijing 100050 (CN); WANG, Xiaoliang, Beijing 100050 (CN); WANG, Xiaojing, Beijing 100050 (CN); MA, Shuanggang, Beijing 100050 (CN); WANG, Ling, Beijing 100050 (CN); LI, Yong, Beijing 100050 (CN); FENG, Nan, Beijing 100050 (CN); LI, Mi, Beijing 100050 (CN); XU, Shaofeng, Beijing 100050 (CN); CAI, Jie, Beijing 100050 (CN); QU, Jing, Beijing 100050 (CN); WANG, Weiping, Beijing 100050 (CN); LIU, Yunbao, Beijing 100050 (CN); ZHANG, Mi, Beijing 100050 (CN)
(74) Representative: Ghirardi, Valeria
(86) International application number: PCT/CN2024/094402
(87) International publication number: WO 2025/081816

(57) **Abstract**

The present invention relates to the technical field of medicines. Provided are keratin CF6, a preparation method therefor, and a pharmaceutical composition and the use thereof. Specifically, the present invention relates to keratin CF6, a nucleic acid molecule encoding keratin CF6, an expression vector comprising the nucleic acid molecule, a host cell comprising the expression vector or genome of which is integrated with the nucleic acid molecule, a method for preparing keratin CF6, a pharmaceutical composition comprising keratin CF6, and the use of the keratin CF6, nucleic acid molecule, expression vector, host cell or pharmaceutical composition described above in the preparation of an antipyretic, analgesic, antitussive and phlegm-expelling, anticonvulsant, antiepileptic, antihypertensive, anti-inflammatory, or antiviral drug.

## Description

### Technical Field

The present invention pertains to the field of medical technology, relates to a keratin CF6, a nucleic acid molecule encoding the keratin CF6, an expression vector comprising the nucleic acid molecule, and a host cell comprising the expression vector or having the nucleic acid molecule integrated into its genome, and a preparation method for the keratin CF6 and a pharmaceutical composition comprising the keratin, and a use of said keratin and the pharmaceutical composition in the preparation of a medicament for antipyretic, analgesia, antitussive expectorant, anticonvulsant, antiepileptic, hypotensive, anti-inflammatory, and antiviral purposes.

### Background Art

Keratin is a kind of protein, which is widely found in the epidermis of humans and animals, and is the main component of hair, feathers, hoofs, shells, claws, horns, etc. It is an extremely important structural protein for connective tissue and plays a role in protecting the body.

Keratin exists widely in organisms and is a kind of renewable resource with great utilization value, but it has not been widely and effectively utilized. The main reason is that keratin is not easy to dissolve in various solvents, and keratin is generally more resistant to enzymatic hydrolysis by proteases than other proteins. Therefore, it is very difficult to extract and prepare natural keratin.

With the rapid development of modern biotechnology such as genomics, proteomics, genetic engineering, microbial engineering, etc., more and more genes are discovered. The use of protein expression systems to prepare and produce target proteins is an important method for studying the biological functions of genes or proteins.

It is novel and innovative and is not reported in other literatures to prepare a target keratin by utilizing a protein expression system, and further to study its structure and function.

### Summary of the Invention

The technical problem solved by the present invention is to provide a keratin CF6, a nucleic acid molecule encoding the keratin CF6, an expression vector comprising the nucleic acid molecule, and a host cell comprising the expression vector or having the nucleic acid molecule integrated into its genome, and a preparation method of the keratin CF6, a pharmaceutical composition comprising the keratin CF6, and use of the above-mentioned keratin CF6, nucleic acid molecule, expression vector, host cell, or pharmaceutical composition in the manufacture of a medicament for antipyretic, analgesia, antitussive, expectorant, anticonvulsant, antiepileptic, hypotensive, anti-inflammatory and antiviral.

To solve the technical problems of the present invention, the present invention provides the following technical solutions:
The first aspect of the technical solution of the present invention is to provide a keratin CF6, characterized in that the amino acid sequence of the keratin CF6 is:
(1) the amino acid sequence set forth in SEQ ID NO.1 in the sequence listing; or
(2) an amino acid sequence formed by substitution, deletion or addition of 1-35 amino acids in the amino acid sequence set forth in SEQ ID NO. 1 in the sequence listing, and that basically maintains the same biological function.

Further, conventional modification can be performed on the keratin CF6; or a tag for detection or purification can be attached to the keratin CF6.

Furthermore, the conventional modification includes acetylation, amidation, cyclization, glycosylation, phosphorylation, alkylation, biotinylation, fluorescent group modification, polyethylene glycol PEG modification, immobilization modification, sulfation, oxidation, methylation, deamination, formation of disulfide bond or disulfide bond breakage; The tag includes His6, GST, EGFP, MBP, Nus, HA, IgG, FLAG, c-Myc, and Profinity eXact.

The second aspect of the technical solution of the present invention provides a nucleic acid molecule encoding the keratin CF6 of the first aspect.

Further, the nucleotide sequence of the nucleic acid molecule is:
(1) the nucleotide sequence set forth in SEQ ID NO. 2 in the sequence listing;
(2) a nucleotide sequence obtained by sequence optimization based on the nucleotide sequence set forth in SEQ ID NO. 2; or
(3) a nucleotide sequence complementary to the nucleotide sequence in (1) or (2) above-mentioned.

The third aspect of the technical solution of the present invention provides an expression vector, characterized in that the expression vector comprises the nucleic acid molecule described in the second aspect.

Further, the expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K, etc.; the preferred expression vector is the pET series vector; the most preferred expression vector is pET-28a(+).

The fourth aspect of the technical solution of the present invention provides a host cell, characterized in that the host cell comprises the expression vector of the third aspect or has the nucleic acid molecule of the second aspect integrated into its genome.

Further, the host cell includes bacterium, yeast, aspergillus, plant cell, or insect cell.

Furthermore, the bacterium includes *Escherichia coli* or yeast.

Competent host cells can be BL21 series, Transetta series, Rosetta series, DH5α series, JM series, Top series, Origami series, Trans1-T1, TG1, TB1; Y11430, MG1003, GS115 (AOX1), KM71, SMD1168, etc.; the preferred competent cells for expression are BL21 (DE3), Transetta (DE3).

The fifth aspect of the technical solution of the present invention provides a method for preparing the keratin CF6 of the first aspect, which is characterized in that it comprises the following steps:
A. synthesizing the nucleic acid molecule corresponding to the keratin CF6 described in the first aspect, ligating the nucleic acid molecule into a corresponding expression vector, transforming the expression vector into a host cell, culturing the host cell comprising the expression vector in fermentation equipment under a certain condition and inducing expression of the keratin CF6 to obtain a crude protein solution containing the keratin CF6;
B. separating, purifying and drying the crude protein solution obtained in step A to obtain the keratin CF6.

Further, in step A, the host cell is mainly selected from *Escherichia coli*, the keratin CF6 is expressed in inclusion bodies of *Escherichia coli*, and the fermentation equipment includes shaker flask or fermentation tank.

Further, in step A, after inducing the expression of the keratin CF6, using a detergent to wash impurities, then using a urea solution to dissolve to obtain a crude protein solution.

Further, a medium that can be used in step A may be LB medium, TB medium, SB medium, SOB medium, SOC medium, PDA medium, YPD medium, rose Bengal medium, salt Czapek Dox medium, DOBA medium, rice koji medium and its modified formula, etc.; for shake flask fermentation, LB medium and TB medium are preferred, with TB medium being the most preferred; for fermentation in a fermentation tank, LB medium and its modified formula are preferred.

Further, an inducer that can be used in step A can be IPTG, lactose, arabinose, etc.; preferably IPTG, lactose.

Further, in step A, centrifuging the obtained fermentation broth and then discarding the supernatant; suspending the precipitate in a buffer, followed by disrupting bacterial cell and subsequent centrifuging to remove the supernatant; washing the precipitate with a detergent, and then dissolving the precipitate in a urea solution to obtain a CF6 crude protein solution.

Among them, the buffer is preferably buffer A, and its dosage is: fermentation broth volume : buffer A volume = 1~100:1, preferably 10:1.

The detergent can be urea solution, guanidine hydrochloride solution, triton and buffer A, etc., preferably urea solution, most preferably 2 M urea solution (which may contain 1% Triton) and 4 M urea solution. The dosage is: fermentation broth volume: urea solution volume = 0.2~100:1, preferably 1~15:1.

The urea solution is preferably a 6 M~8 M urea solution, most preferably an 8 M urea solution and its dosage is: fermentation broth volume: 8 M urea volume = 0.2~100: 1, preferably 2~15: 1.

Further, in step B, the method for separating and purifying includes ultrafiltration, or microfiltration membrane technology purification method, column chromatography purification method, salting-out method, and dialysis method.

Further, in step B, the method for separating and purifying is as follows:
(1) The dialysis method is to purify the crude protein solution obtained in step A by a dialysis method to obtain the target protein CF6 solution.

The molecular weight cut-off of a dialysis bag can be 0.5-10 kD, the preferred molecular weight cut-off of a dialysis bag is 3.5-10 kD, and the most preferred molecular weight cut-off of a dialysis bag is 10 kD.

(2) The method for ultrafiltration and microfiltration is to purify the crude protein solution obtained in step A by membrane technology, such as an ultrafiltration membrane or microfiltration membrane, to obtain a concentrated solution of the target protein CF6.

(3) The column chromatography method is to pass the crude protein solution obtained in step A through column chromatography, such as various exchange columns or exclusion column chromatography, to separate and purify the target protein CF6.

The preferred exclusion column is dextran gel column, Superdex 30 Increase, Superdex 75 Increase, Superdex 200 Increase, Superose 6 Increase, etc.;

The preferred exchange column is an ion exchange resin column: anion exchange resin column: HiTrap Q FF, HiTrap Capto Q ImpRes, Capto Q ImpRes, HiTrap Capto Q, HiTrap DEAE, Toyopearl Q-650M, Toyopearl SuperQ-650M, etc.; Cation exchange resin column: HiTrap SP FF, HiTrap Capto SP ImpRes, Capto SP ImpRes, HiTrap Capto SP, Toyopearl SP-650M, Toyopearl Super SP-650M.

As an eluent, commonly used eluents in the art can be used, such as water, salt solution. The salt solution includes sodium chloride solution, sodium dihydrogen phosphate solution, disodium hydrogen phosphate solution, sodium acetate, acetic acid, and the like.

(4) The salting-out method is to purify the crude protein solution obtained in step A by salting-out method to obtain the target protein CF6 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

Further, the target protein CF6 solution purified in step B can be freeze-dried or vacuum-dried into a dry powder, or can be directly spray-dried from a concentrated liquid into a dry powder.

The sixth aspect of the technical solution of the present invention provides a pharmaceutical composition, characterized in that the pharmaceutical composition comprises the keratin CF6 described in the first aspect or the nucleic acid molecule described in the second aspect or the expression vector described in the third aspect or the host cell described in the fourth aspect and a pharmaceutically acceptable carrier or excipient.

The keratin obtained in the above-mentioned steps of the present invention can be freeze-dried or vacuum-dried into a dry powder, or can be directly spray-dried from a concentrated liquid into a dry powder, and then made into various dosage forms.

The present invention relates to a pharmaceutical composition, which comprises any keratin obtained in the above-mentioned steps and a pharmaceutically acceptable carrier.

The present invention also relates to a pharmaceutical composition comprising the keratin of the present invention as an active ingredient and a conventional pharmaceutical excipient or adjuvant. Generally, the keratin of the present invention accounts for 0.1-100.0% of the total weight of the pharmaceutical composition.

The present invention also provides a pharmaceutical composition, which comprises a pharmaceutical effective dose of protein as an active ingredient and a pharmaceutically acceptable carrier.

The pharmaceutical composition of the present invention can be prepared according to methods known in the art. When used for this purpose, if necessary, the protein of the present invention can be combined with one or more solid or liquid pharmaceutical excipients and/or adjuvants to prepare an appropriate administration form or dosage form that can be used as human drugs or veterinary drugs.

The keratin of the present invention or a pharmaceutical composition comprising the same can be administered in a unit dosage form. The route of administration can be enteral or parenteral, such as oral, intramuscular, subcutaneous, nasal, oral mucosal, ocular, pulmonary, transdermal, vaginal, peritoneal, and rectal, etc., oral administration is preferred.

The keratin protein of the present invention or a pharmaceutical composition comprising the same can be administered by injection. Injections include intravenous injection, intramuscular injection, subcutaneous injection, intradermal injection, intraperitoneal injection, and acupoint injection, etc.

The dosage form for administration may be a liquid dosage form, a solid dosage form, or a semi-solid dosage form. Liquid dosage forms can be solutions (including true solutions and colloidal solutions), emulsions (including oil-in-water, water-in-oil, and multiple emulsions), suspensions, injections (including water injections, powder injections, and infusions), eye drops, nasal drops, lotions, and liniments, etc. The solid dosage form can be tablets (including ordinary tablets, enteric-coated tablets, buccal tablets, dispersible tablets, chewable tablets, effervescent tablets, orally disintegrating tablets), capsules (including hard capsules, soft capsules, and enteric-coated capsules), granules, powders, pellets, dripping pills, suppositories, films, patches, air (powder) sprays, sprays, etc.; semi-solid dosage forms can be ointments, gels, pastes, etc.

The keratin of the present invention can be made into ordinary preparations, slow-release preparations, controlled-release preparations, targeted preparations, and various particle delivery systems.

To make a unit administration dosage form into a tablet, various excipients known in the art can be widely used, including diluents, binders, wetting agents, disintegrants, lubricants, glidants. The diluent can be starch, dextrin, sucrose, glucose, lactose, mannitol, sorbitol, xylitol, microcrystalline cellulose, calcium sulfate, calcium hydrogen phosphate, calcium carbonate, etc.; the wetting agents can be water, ethanol, isopropanol, etc.; the binder can be starch slurry, dextrin, syrup, honey, glucose solution, microcrystalline cellulose, acacia slurry, gelatin slurry, sodium carboxymethyl cellulose, methyl cellulose, hydroxypropyl methyl cellulose, ethyl cellulose, acrylic resin, carbomer, polyvinylpyrrolidone, polyethylene dipropyl alcohol, etc.; the disintegrant can be dry starch, microcrystalline cellulose, low-substituted hydroxypropyl cellulose, cross-linked polyvinylpyrrolidone, cross-linked sodium carboxymethyl cellulose, sodium carboxymethyl starch, sodium bicarbonate and citric acid, calcium carbonate, polyoxyethylene sorbitol fatty acid ester, dodecyl sodium sulfonate; the lubricants and glidants can be talc, silicon dioxide, stearate, tartaric acid, liquid paraffin, polyethylene glycol, etc.

The tablets can also be further made into coated tablets, such as sugar-coated tablets, film-coated tablets, enteric-coated tablets, or double-layer tablets and multi-layer tablets.

To make the administration unit into a pill, various carriers known in the field can be widely used. Examples of carriers are, for example, diluents and absorbents, such as glucose, lactose, starch, cocoa butter, hydrogenated vegetable oil, polyvinylpyrrolidone, polyethylene glycol laurate, kaolin, talc, etc.; binders, such as Gum arabic, xanthan gum, gelatin, ethanol, honey, liquid sugar, rice paste or batter, etc.; disintegrants, such as agar powder, dried starch, alginate, sodium lauryl sulfonate, methyl cellulose, ethyl cellulose, etc.

To make the administration unit into a suppository, various carriers known in the field can be widely used. Examples of carriers are, for example, polyethylene glycol, lecithin, cocoa butter, higher alcohols, higher alcohol esters, gelatin, semi-synthetic glycerides, and the like.

To make the administration unit into a capsule, the keratin of the present invention as an active ingredient is mixed with the above-mentioned various carriers, and the resulting mixture is placed into hard gelatin capsules or soft capsules. The keratin of the present invention as an active ingredient may also be formulated as microcapsules, suspended in an aqueous medium to form a suspension, or filled into hard capsules or prepared as injections for administration.

For example, the keratin of the present invention is prepared into injection preparations, such as solutions, suspension solutions, emulsions, and freeze-dried powder injections. Such preparations may be aqueous or non-aqueous and may contain one and/or more pharmacodynamically acceptable carriers, diluents, binders, lubricants, preservatives, surfactants, or dispersants. For example, the diluents can be selected from water, ethanol, polyethylene glycol, 1,3-propylene glycol, ethoxylated isostearyl alcohol, polyoxylated isostearyl alcohol, polyoxyethylene sorbitol fatty acid esters and the like. In addition, in order to prepare an isotonic injection, an appropriate amount of sodium chloride, glucose or glycerin can be added to the injection preparation. In addition, conventional auxiliary solvents, buffers, pH adjusters, etc. can also be added. These auxiliary materials are commonly used in this field.

In addition, if necessary, coloring agents, preservatives, flavors, corrigents, sweeteners or other materials can also be added to the pharmaceutical preparations.

In order to achieve the purpose of medication and enhance the therapeutic effect, the keratin or the pharmaceutical composition of the present invention can be administered by any known administration method.

The administration dosage of the keratin pharmaceutical composition of the present invention depends on many factors, such as the nature and severity of the disease to be prevented or treated, the gender, age, weight, personality and individual response of the patient or animal, the route of administration, the frequency of administrations and the purpose of treatment, therefore the therapeutic dose of the present invention can have a wide range of changes. Generally speaking, the dosage of the pharmaceutical ingredients of the present invention is well known to those skilled in the art. Appropriate adjustments can be made according to the actual amount of the drug contained in the final preparation of the keratin composition of the present invention to meet the requirement of the therapeutically effective amount and accomplish the preventive or therapeutic purpose of the present invention. The appropriate daily dosage range of the keratin of the present invention: the dosage of the keratin of the present invention is from 0.01 to 500 mg/kg body weight, preferably from 0.5 to 100 mg/kg body weight, more preferably from 1 to 50 mg/kg body weight, and most preferably from 2 to 30 mg/kg body weight. The above dosage can be administered in a single dosage form or divided into several, such as two, three, or four dosage forms for administration, depending on the clinical experience of the administering doctor and the dosage regimens, including the use of other treatments. The total dose required for each treatment can be divided into multiple or single doses for administration. The protein or pharmaceutical composition of the present invention can be administered alone, or be administered in combination with other therapeutic drugs or symptomatic drugs with adjusting the dose.

The seventh aspect of the technical solution of the present invention provides a use of the keratin CF6 according to the first aspect or the nucleic acid molecule according to the second aspect or the expression vector according to the third aspect or the host cell according to the fourth aspect or the pharmaceutical composition according to the sixth aspect in the manufacture of a medicament for antipyretic, analgesia, antitussive, expectorant, anticonvulsant, antiepileptic, hypotensive, anti-inflammatory, and antiviral.

To accomplish the purpose of the present invention, the present invention takes the following technical solutions. Specifically, the preparation of the keratin CF6 of the present invention includes the following steps:
(1) synthesizing a polynucleotide and determining the accuracy of the sequence thereof;
   the preferred polynucleotide is of the sequence set forth in SEQ ID No. 2;
(2) inserting the polynucleotide into an expression vector;
   the expression vector can be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K etc.; the preferred expression vector is the pET series; the most preferred expression vector is pET-28a(+);
(3) transfecting the expression vector into a host cell;
   the host cell can be *E. coli* or yeast; the preferred host cell is *E. coli*;
   competent cells can be BL21 series, Transetta series, Rosetta series, DH5α series, JM series, Top series, Origami series, Trans1-T1, TG1, TB1; Y11430, MG1003, GS115 (AOX1), KM71, SMD1168, etc. The preferred competent cells for expression are BL21(DE3) and Transetta (DE3);
(4) performing fermentation culture of the host cell to induce the expression of the target protein CF6 under an appropriate condition;
   fermentation equipment can use a shake flask or a fermentation tank;
   the medium can be LB medium, TB medium, SB medium, SOB medium, SOC medium, PDA medium, YPD medium, rose Bengal medium, salt Czapek Dox medium, DOBA medium, rice koji culture medium and their improved formulas, etc.; for shake flask fermentation, LB medium and TB medium are preferred, with TB medium being the most preferred; for fermentation in a fermentation tank, LB medium and its improved formulas are preferred;
   an inducer can be IPTG, lactose, arabinose, etc.; the preferred ones are IPTG, lactose;
(5) enriching the target protein CF6;
   centrifuging the fermentation broth obtained in step (4), and discarding the supernatant; suspending the precipitate in a buffer, followed by disrupting bacterial cell and subsequent centrifuging to remove the supernatant; washing the precipitation with a detergent, then dissolving the precipitation in a urea solution to obtain a CF6 crude protein solution;
   among them, the buffer is preferably buffer A, and its dosage is: fermentation broth volume: buffer A volume = 1~100: 1, preferably 5~10: 1;
   the detergent can be urea solution, guanidine hydrochloride solution, Triton, buffer A, etc., preferably urea solution, most preferably 2 M urea solution (which may contain 1% Triton), the dosage is fermentation broth volume: urea solution volume = 0.2~100: 1, preferably 1~15: 1; the washing procedure is performed 3~10 times, preferably 6 times;
   the urea solution is preferably a 6 M~8 M urea solution, most preferably an 8 M urea solution, its dosage is: fermentation broth volume: 8 M urea solution volume = 0.2~100:1, preferably 2~15: 1;
(6) separating and purifying the target protein CF6.

The crude protein solution obtained in step (5) needs to be purified to obtain the target protein CF6. The purification may be carried out by dialysis, or ultrafiltration and microfiltration, or column chromatography, or salting-out steps.
A. The dialysis step is to purify the crude protein solution obtained in step (5) by a dialysis method to obtain the target protein CF6 solution.
   The molecular weight cut-off of a dialysis bag can be 0.5-10 kD, the preferred molecular weight cut-off of a dialysis bag is 3.5-10 kD, and the most preferred molecular weight cut-off of a dialysis bag is 10 kD.
B. The step of ultrafiltration and microfiltration is to purify the crude protein solution obtained in step (5) by membrane technology, such as an ultrafiltration membrane or microfiltration membrane, to obtain the target protein CF6 concentrated solution.
C. The column chromatography step is to pass the crude protein solution obtained in step (5) through a column chromatography, such as various exchange columns or exclusion column chromatography, to separate and purify and to obtain the target protein CF6.

The preferred exclusion column is a dextran gel column, Superdex 30 Increase, Superdex 75 Increase, Superdex 200 Increase, Superose 6 Increase, etc.; the preferred exchange column is an ion exchange resin column: anion exchange resin column, HiTrap Q FF, HiTrap Capto Q ImpRes, Capto Q ImpRes, HiTrap Capto Q, HiTrap DEAE, Toyopearl Q-650M, Toyopearl SuperQ-650M, etc.; a cation exchange resin column, HiTrap SP FF, HiTrap Capto SP ImpRes, Capto SP ImpRes, HiTrap Capto SP, Toyopearl SP-650M, Toyopearl Super SP-650M. The most preferred is an anion exchange resin column.

As an eluent, commonly used eluents in the art can be used, such as water, salt solution, the salt solution includes sodium chloride solution, sodium dihydrogen phosphate solution, disodium hydrogen phosphate solution, sodium acetate, acetic acid, and the like.

D. The salting-out step is to purify the crude protein solution obtained in step (5) by a salting-out method to obtain the target protein CF6 suspension.

The salting-out agent can be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of times of salting out is 1 to 3 times, preferably 2 times.

After salting out, the precipitate is washed by adding pure water for 2 to 5 times, preferably 3 times.

The target protein CF6 solution purified from steps A to D can be freeze-dried or vacuum-dried into dry powder, or can be directly spray-dried from a concentrated solution into dry powder.

The beneficial technical effects of the present invention:
1. The protein of the present invention is a keratin obtained for the first time, and the preparation method of the present invention has the characteristics of high yield and high sample purity.
2. By studying the pharmacodynamics of the protein CF6 on the yeast-induced SD rat fever model in the invention, it proves that the protein CF6 (10 mg/kg) has a significant effect in reducing the elevated body temperature of the model animals at 4 hours post-modeling.
3. This invention, through an efficacy test of protein CF6 on pentylenetetrazol (PTZ)-induced epilepsy in mice, demonstrates that protein CF6 (200 mg) can significantly prolong the latency of grade III epileptic seizures in mice.
4. The present invention, through a pharmacodynamic study of protein CF6 using the ammonia-induced cough model in mice, demonstrates that protein CF6 (20 mg/kg) reduces cough frequency and exhibits an antitussive effect.
5. The present invention, through a pharmacodynamic study of protein CF6 using the phenol red excretion method in mice, confirms that protein CF6 at both doses (20 mg/kg and 50 mg/kg) has a significant expectorant effect.

### Brief Description of the Drawings

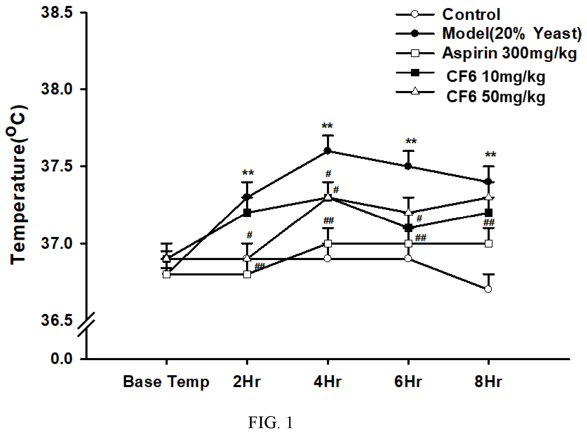

FIG. 1: Effect of protein CF6 on yeast-induced fever model in rats.

(Compared with normal control group, ** P<0.01; Compared with the model group, # P<0.05, ## P<0.01)

### EXAMPLES

The following examples and pharmacological activity test examples are used to further illustrate the present invention, but this does not mean any limitation to the present invention.

The experimental methods in the following examples and pharmacological activity test examples are conventional methods unless otherwise specified; the experimental materials used, unless otherwise specified, are purchased from conventional biochemical reagent companies.

### Example 1 Preparation of protein CF6 crude solution A by shake flask fermentation (TB medium)

The nucleotide sequence shown in SEQ ID No.2 was synthesized and transferred into the pET-28a(+) vector. It was confirmed by sequencing that an expression vector comprising the correct sequence was obtained. And the expression vector was transfected into BL21(DE3) cells and expression competent host cells comprising the target nucleotide sequence were obtained. The expression competent host cells were added into LB medium and cultured in a shaker at 37°C and 220 rpm for 1 hour to obtain a recombinant strain.

The recombinant strain was dipped and streaked in LBA plates containing Kanamycin. The plates were placed upside down in a 37°C constant temperature incubator and incubated overnight for 16 hours.

400 mL of TB medium was prepared and divided into 2 bottles, 200 mL in each bottle. Kanamycin (final concentration 50 µg/mL) was added to each bottle (200 mL) of TB medium. A single colony on the plate was taken and added to the TB medium and amplified and cultured overnight at 37°C and 220 rpm in the shaker to obtain seed liquid.

28.8 L of TB medium was prepared and divided into 144 bottles, 200 mL in each bottle. Each bottle (200 mL) of TB medium was added with Kanamycin (final concentration 50 µg/ml), then added with 2 mL of the seed liquid, and cultured in a shaker at 37°C and 220 rpm for 2-3 hours. The OD₆₀₀ was monitored, when the OD₆₀₀ reached about 1.0, an inducer was added to induce protein expression in a shaker, and the induction conditions were selected from the following table.

| | Inducer | Induction temperature | Induction time | Shaker speed |
|---|---|---|---|---|
| Inducing conditions | IPTG (Final concentration 0.5 mM) | 16 °C | 16 h | |
| | | 25 °C | 8 h | 220 rpm |
| | | 37 °C | 5 h | |

All bottles of bacterial liquid were combined, centrifuged at 7000 rpm for 5 minutes, and the supernatant was discarded after sterilization. The precipitate was suspended in about 3 L of buffer, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes, the supernatant was discarded, and the precipitate (i.e. inclusion body) was obtained. The precipitate was washed with detergent (three times with 1 L of 2 M urea - 1% Triton solution, twice with 1 L of 2 M urea solution), centrifuged, and the supernatant was discarded. The precipitate was then dissolved in 1 L of 8 M urea solution, and the crude protein solution A was obtained.

### Example 2 Preparation of protein CF6 crude solution B (other medium) by fermentation in a fermentation tank

In Example 1, an expression vector was synthesized and sequenced to confirm that the expression vector comprising the sequence shown in SEQ ID No. 2 was obtained. The expression vector was transfected into BL21 (DE3) cells and expression-competent host cells comprising the target nucleotide sequence were obtained.

20 mL of LB medium was prepared and 50 µL of host cells comprising the target nucleotide sequence were added into 800 µL of LB medium and were cultured in a shaker at 37 °C and 220 rpm for 1 hour.

The above bacterial liquid was dipped and streaked into an LBA plate containing Kanamycin, and then the plates were placed upside down and cultured in a constant temperature incubator at 37°C overnight for 16 hours.

10 mL of LB medium was obtained and added with Kanamycin (final concentration 50 µg/mL). Single colonies were taken, added into LB medium and were cultured overnight at 37°C and 220 rpm for 15 hours, then the seed solution was obtained.

1 L of the medium as indicated in the table below was prepared and divided it into 10 bottles, 100 mL each. Kanamycin (final concentration 50 µg/mL) was added to each bottle (100 mL) of medium, and 1 mL of seed solution was added and cultured at 37°C and 220 rpm for 2-3 hours in a shaker. OD₆₀₀ was monitored, and when OD₆₀₀ reached about 1.0, the inducer IPTG (final concentration 0.5 mM) was added. And the protein expression was induced at 37°C and 220 rpm in a shaker.

| | |
|---|---|
| Culture medium | LB medium, SOB medium, SOC medium |

Each bottle of bacterial liquid was combined and was centrifuged at 10000 rpm for 10 minutes, and the supernatant was discarded after sterilization. The precipitate was suspended into about 100 mL of buffer, filtered with an 80-100 mesh screen, and the filtrate was crushed with a high-pressure crusher at a pressure of 800-1000 bar, twice, 2 minutes each time. The broken bacteria liquid was centrifuged at 10000 rpm for 30 minutes and the supernatant was discarded.

The precipitate was firstly added with 40 mL of detergent buffer A, washed and centrifuged, and the supernatant was discarded. Then, the resulting precipitate was added with 40 mL of wash solution containing 2 M urea (with 1% Triton), washed twice and centrifuged, and the supernatant was discarded each time. Subsequently, the precipitate was added with 40 mL of 2 M urea solution, washed twice and centrifuged, and the supernatant was discarded after each wash. Finally, the resulting precipitate was added with 40 mL of 8 M urea solution, and the crude protein solution B was obtained.

### Example 3 Preparation of protein CF6 crude solution C by fermentation in a fermentation tank

In Example 1, an expression vector was synthesized and sequenced to confirm that the expression vector comprising the sequence shown in SEQ ID No. 2 was obtained. The expression vector was transfected into BL21 (DE3) cells and expression-competent host cells comprising the target nucleotide sequence were obtained. The expression competent host cells were added into LB medium and cultured in a shaker at 37°C and 220 rpm for 1 hour to obtain a recombinant strain.

100 µl of the recombinant strain was added to an LBA plate containing Kanamycin, spread evenly with a spreader until dry, and the plate was placed upside down in a constant temperature incubator at 37°C for overnight culture. Three single colonies were taken separately, streaked on plates containing Kanamycin, and then the plates were cultured overnight. After the verifications of three batches of shake flask fermentation and expression were confirmed to be correct, the strains were preserved with 15% glycerol and were divided into 0.8 mL for each to obtain a working cell bank, which was stored in a refrigerator at -80°C for later use.

100 µL of one glycerol bacteria stock was taken out from the working cell bank, added with 40 mL LB medium, added with Kanamycin (final concentration 50 µg/mL), and cultured in a shaker at 37°C and 220 rpm for 6 hours to obtain a first-level seed liquid.

1.2 mL of the first-level seed liquid was taken, added to 120 mL of LB medium, added with Kanamycin (final concentration 50 µg/mL), and then cultured in a shaker at 37°C and 220 rpm for 6 hours to obtain a second-level seed liquid.

3 L of modified LB medium was added to a 5 L fermentation tank, then added with 120 mL of the second-level seed liquid and 3 mL of Kanamycin (final concentration 50 µg/mL), and cultured at 37°C and 30% dissolved oxygen (series speed) for about 8 hours. The OD value was monitored to be about 20, and 3 g lactose was used as an inducer. Induction was performed at 20°C, fed at a rate of 30 mL/hour, and cultured at 20°C for 24 hours.

The bacteria liquid was centrifuged at 7000 rpm for 5 minutes, and the supernatant was discarded after sterilization. The precipitate was suspended in about 600 mL of buffer A, filtered with an 80-100 mesh screen, and the filtrate was crushed with a high-pressure crusher at a pressure of 800-1000 bar, twice, 2 minutes each time. The broken bacteria liquid was centrifuged at 7000 rpm for 30 minutes and the supernatant was discarded.

The precipitate was added with 2 M urea solution (containing 1% Triton) and washed 3 times, 1 L each time. Then the precipitate was added with 1 L of 2 M urea solution, washed twice and centrifuged, and the supernatant was discarded. Subsequently, the precipitate was added with 1 L of 4 M urea solution, washed one time and centrifuged, and the supernatant was discarded. Finally, the precipitate was added with 2 L of 8 M urea solution, and the crude protein solution C was obtained.

### Example 4 Preparation of protein CF6 from crude protein solution A by dialysis

Protein crude solution A obtained in Example 1 was filtered with a 0.45 µm filter membrane, and the filtrate was combined. The filtrate was dialyzed with water, the molecular weight cutoff of the dialysis bag was 10 kD. After 72 hours of dialysis, the inner liquid was freeze-dried to obtain the target protein CF6.

### Confirmation of the structure of protein CF6 by LC-MS/MS-based complete protein sequence analysis

Main materials: Acetonitrile, formic acid, ammonium bicarbonate, dithiothreitol (DTT), iodoacetamide (IAA), trypsin, chymotrypsin, Glu-C, Asp-N;
Main instruments: Capillary High Performance Liquid Chromatograph (Thermo Ultimate 3000), Electrospray-Combined Ion Trap Orbitrap Mass Spectrometer (Thermo Q Exative Hybrid Quadrupole-Orbitrap Mass Spectrometer).

### Methods and results:

Protein CF6 was subjected to pretreatment such as dissolution replacement, reductive alkylation, and various proteolysis to obtain enzyme-cleaved peptides. The solution of enzyme-cleaved peptides was analyzed by liquid chromatography tandem mass spectrometry. The original mass spectrometry file was retrieved from the protein database using Maxquant (1.6.2.10) for analysis data. The identification results determined that it was consistent with the target sequence of SEQ ID No.1.

### Example 5 Preparation of protein CF6 by purification from protein crude solution A through salting-out method

The protein crude solution A was placed in a stirred container for salting-out, twice. Saturated ammonium sulfate solution was slowly added along the wall to make the final concentration of ammonium sulfate reach 25% or 50%. During the salting-out process, the protein was separated out. After the salting-out was completed, the solution was then filtered, and the first salting-out was completed. Then 400 ml of pure water was added into the precipitate to suspend, and a saturated solution of ammonium sulfate was slowly added along the wall again to make the final concentration of ammonium sulfate reach 25%. Then the second salting-out and filtration were carried out, and the precipitate was the crude protein extract. The crude protein extract was washed three times with water: 200 mL of pure water was added to suspend, stirred, left standing, and filtered. After repeated three times, the resulting precipitate was then freeze-dried to obtain the target protein CF6.

The product protein CF6 was confirmed to have the same amino acid sequence as that of the protein prepared in Example 4 through the same structural confirmation method as in Example 4.

### Example 6 Preparation of protein CF6 by purification from the protein crude solution B

The protein crude solution B obtained in Example 2 was purified by the following two methods:
The first method: dialysis;
The crude protein solution B was filtered with a 0.45 µm membrane, the filtrate was dialyzed with water, for more than 72 hours, and the inner solution was freeze-dried to obtain the target protein CF6.

| | |
|---|---|
| Dialysis bag | Molecular weight cutoff:0.5 kD, 3.5 kD, 5 kD, 10 kD |

The second method: salting out;
The crude protein solution B was placed in a stirred container for salting-out twice. Saturated ammonium sulfate solution was slowly added along the wall to make the final concentration of ammonium sulfate reach 25% or 50%. During the salting-out process, the protein was separated out. After the salting-out was completed, the solution was filtered, and the first salting-out was completed. 400 mL of pure water was added into the precipitate to suspend, and then a saturated solution of ammonium sulfate was slowly added along the wall again to male the final concentration of ammonium sulfate reach 25%. The second salting-out and filtration were carried out, and the precipitate was the crude protein extract. The crude protein extract was washed three times with water: 200 mL of pure water was added to suspend, stirred, left standing, and filtered. After repeated three times, the resulting precipitate was then freeze-dried to obtain the target protein CF6.

The product protein CF6 obtained by the two methods was confirmed to have the same amino acid sequence as that of the protein prepared in Example 4 through the same structural confirmation method as in Example 4.

### Example 7 Preparation of protein CF6 by purification from crude protein solution C.

Crude protein solution C was purified by microfiltration membrane technology: 20 nm or 50 nm ceramic membrane core was used for repeated microfiltration to remove urea. The inner liquid was freeze-dried to obtain the target protein CF6.

The product protein CF6 was confirmed to have the same amino acid sequence as that of the protein prepared in Example 4 through the same structural confirmation method as in Example 4.

### Pharmacological test

### Experimental example 1 The pharmacodynamic test of protein CF6 (the protein in Example 7) on yeast-induced fever model in SD rats

Animals: male SD rats, weight: 230-260 grams;
Drugs: yeast (OXOID LP0021), aspirin (SIGMA A2093), protein CF6;
Instruments: electronic balance (SARTORIUS BP121S), electronic clinical thermometer (CITIZEN CT-513W).

### Experiment grouping:

Normal control group;
Model group: yeast-induced fever model group;
Positive control group: Aspirin 300 mg/kg group;
Protein CF6, 10 mg/kg group, 50 mg/kg group.

### Method:

Preparation of experimental animals**:** After 1 day of acclimatization in the experimental environment (temperature 22°C ± 2°C, relative humidity 50% ± 2%), pre-adaptation rectal temperature measurements were performed at 8:00 AM and 3:00 PM. Animals were fasted for 12 hours with free access to water prior to experiments. Before temperature measurement, animals were allowed to defecate. The probe of the digital thermometer was coated with petroleum jelly and inserted 2 cm into the rat rectum (marked at 2 cm to ensure consistent depth). Temperature readings were recorded after stabilization.

Subcutaneous injection of dry yeast-induced rat fever model: Basal body temperature was measured before modeling. Rats with temperatures between 36.2-37.3°C were selected as qualified subjects and randomly divided into groups (n=8 per group). Aspirin or varying doses of protein CF6 were administered orally, immediately followed by subcutaneous injection of 20% yeast suspension (10 mL/kg). The normal control group received an equal volume of saline subcutaneously. Temperature monitoring commenced 2 hours post-modeling and continued at 2-hour intervals for 8 hours.

### Data Statistics:

Mean body temperature ± standard error (SEM) was calculated for each group at all time points. Intergroup comparisons were performed using t-test, with P<0.05 considered statistically significant.

### Experimental results:

Oral administration of aspirin (300 mg/kg) or protein CF6 (10 mg/kg, 50 mg/kg) was followed by immediate subcutaneous injection of 20% yeast suspension to establish the model. Body temperatures were monitored at 2, 4, 6, and 8 hours after modeling. Results are presented in Table 1 and FIG. 1.

**Table 1. Effects of the tested drugs on the yeast-induced fever model in rats**

| Group | N | Basal body temperature (°C) | Body temperature 2 hours after modeling(°C) | Body temperature 4 hours after modeling(°C) | Body temperature 6 hours after modeling(°C) | Body temperature 8 hours after modeling(°C) |
|---|---|---|---|---|---|---|
| Normal control group | 8 | 36.9±0.1 | 36.9±0.1 | 36.9±0.1 | 36.9±0.1 | 36.7±0.1 |
| Model group | 8 | 36.8±0.1 | 37.3±0.1** | 37.6±0.1** | 37.5±0.1** | 37.4±0.1** |
| Positive control group | 8 | 36.8±0.04 | 36.8±0.1## | 37.0±0.1## | 37.0±0.1## | 37.0±0.1## |
| CF6 10 mg/kg | 8 | 36.9±0.1 | 37.2±0.1 | 37.3±0.1# | 37.1±0.1# | 37.2±0.1 |
| CF6 50 mg/kg | 8 | 36.9±0.05 | 36.9±0.1# | 37.3±0.1# | 37.2±0.1 | 37.3±0.1 |

| | | | | | | |
|---|---|---|---|---|---|---|
| (Compared with the normal control group, ** P<0.01; compared with the model group, # P<0.05, ## P<0.01) | | | | | | |

### Experimental results:

Following oral administration of aspirin (300 mg/kg) or protein CF6 (10 mg/kg, 50 mg/kg), a fever model was immediately established by subcutaneous injection of 20% yeast suspension. Body temperature was monitored at 2, 4, 6, and 8 hours post-modeling. The results demonstrated that:
1) The model group exhibited significantly elevated body temperatures at 2, 4, 6, and 8 hours post-modeling compared to the normal control group (*P* < 0.05), indicating statistically significant differences. This confirms the successful establishment of a stable and reliable model.
2) The positive control aspirin group effectively suppressed the yeast-induced temperature increase in model rats at all monitored time points (2, 4, 6, and 8 hours after modeling). Statistically significant differences were observed versus the model group (*P* < 0.05), demonstrating consistent efficacy of the positive control drug aspirin.
3) The 10 mg/kg dose group of protein CF6 effectively inhibited the rise in body temperature in model rats at 4 and 6 hours after modeling, while the 50 mg/kg dose group showed significant inhibition at 2 and 4 hours after modeling. Compared with the model group, the differences were statistically significant (P < 0.05).

### Experimental example 2 Efficacy test of protein CF6 (the protein in Example 7) on pentylenetetrazole (PTZ)-induced epilepsy in mice

Animals: male ICR mice;
Drugs: Pentylenetetrazol (PTZ), Diazepam, Protein CF6.

### Experiment grouping:

Model group;
Diazepam 2 mg/kg group;
Protein CF6, 50 mg/kg group, 200 mg/kg group;

### Method:

### Model preparation and administration:

On the afternoon of the day before modeling, the test drug was administered once. On the day of modeling, the test drug was administered via oral gavage, followed one hour later by an intraperitoneal injection of PTZ (65 mg/kg, modeling agent). The positive control drug was administered once, half an hour before modeling. Animals were continuously observed for 15 minutes following PTZ injection.

Observation index: ① Seizure condition: Onset time of grade III to grade VI; ② Death situation:
Seizure Grading Scale: Based on the Racine Scale: Grade 0: No observable response; Grade I: Facial muscle twitching or rhythmic movement of the mouth corners; Grade II: Head nodding; Grade III: Unilateral limb clonus; Grade IV: Tonic or generalized limb convulsions; Grade V: Generalized tonic-clonic seizures (full-body convulsive seizure).

### Data processing:

The number of seizure and death incidents observed in each group of mice during the experiment, as well as the Grade III and IV latency periods, were recorded. The latency of mice that did not progress to Grade IV seizures was recorded as the maximum value of 900 seconds. The incidence data were statistically analyzed using the Chi-square test. Latency periods are presented as mean ± standard error of the mean (SEM). Inter-group comparisons were performed using the t-test between the model group and other groups. Statistical significance was defined as *P* < 0.05. Experimental results: see Table 2 and Table 3.

**Table 2. Test drug on PTZ-induced epilepsy in mice-statistics of incidents**

| Group | Number of experimental examples | Number of Grade IV incidents | Grade IV seizure rate | Number of deaths | mortality rate |
|---|---|---|---|---|---|
| Model group | 9 | 7 | 78% | 0 | 0 |
| Diazepam 2 mg/kg | 8 | 0** | 0 | 0 | 0 |
| CF6 50 mg/kg | 9 | 6 | 67% | 0 | 0 |
| CF6 200 mg/kg | 9 | 8 | 89% | 0 | 0 |

| | | | | | |
|---|---|---|---|---|---|
| (Compared with the model group, *P<0.05, **P<0.01) | | | | | |

**Table 3. Test drug on PTZ-induced epilepsy in mice-latency of grade III and IV seizures (mean±SEM)**

| Group | Grade III seizure latency (s) | Grade IV seizure latency (s) | Grade |
|---|---|---|---|
| Model group | 63±3 | 302±118 | 3.8±0.1 |
| Diazepam 2 mg/kg | 187±26** | 900±0** | 3.0±0.0** |
| CF6 50 mg/kg | 72±4 | 405±127 | 3.8±0.2 |
| CF6 200 mg/kg | 75±4* | 220±91 | 3.9±0.1 |

| | | | |
|---|---|---|---|
| (Compared with the model group, *P<0.05, **P<0.01) | | | |

### Experimental results:

1) Results demonstrated that in the model group, the Grade IV seizure incidence was 78% with a mortality rate of 0%, indicating successful model establishment.
2) The positive control drug significantly prolonged the latency of both Grade III and Grade IV seizures in mice.
3) In the comparison of latency to Grade III seizures, the 200 mg/kg dose group of CF6 showed a significantly prolonged latency period, with a statistically significant difference compared to the model group.

### Experimental example 3 The pharmacodynamic test of protein CF6 (the protein in Example 7) of antitussive effect on ammonia water-induced cough in mice.

Animals: male ICR mice;
Drugs and reagents: dextromethorphan hydrobromide, ammonia, 0.2% CMC-Na, protein CF6;
Apparatus: Compressed nebulizer (403T), balance (XS105DU).

### Experiment grouping:

Solvent control group;
Dextromethorphan 15 mg/kg group;
Protein CF6, 20 mg/kg group, 50 mg/kg group.

### Method:

### Model establishment and drug administration:

Mice were orally administered dextromethorphan or varying doses of protein CF6 (administration volume: 10 mL/kg). The solvent control group were administered an equal volume of distilled water. After 1 hour, mice were placed in a sealed chamber and exposed to nebulized 10% ammonia solution for 10 seconds. The cough latency and number of coughs within 2 minutes were then recorded.

### Data processing:

The following parameters were documented: oral administration time point, nebulization exposure time point, cough latency (defined as the time interval in seconds from ammonia nebulization onset to the occurrence of first cough), and cough frequency within 2 minutes. A valid cough was identified by simultaneous thoracic contraction and mouth opening. Data are presented as mean ± SEM. Intergroup comparisons between the model group and other groups were performed using t-test, with P<0.05 considered statistically significant.

### Experimental results:

Pretreatment with dextromethorphan (15 mg/kg) or protein CF6 (20 mg/kg, 50 mg/kg) was administered 1 hour before placing mice in a sealed chamber for 10-second exposure to nebulized 10% ammonia solution. The cough latency and cough frequency within 2 minutes were recorded. Results are shown in Table 4.

**Table 4. Antitussive effect experiment of tested drugs on mice cough induced by ammonia water (X+SEM)**

| Group | N | Latency (s) | P | Number of coughs | P |
|---|---|---|---|---|---|
| Solvent control group | 9 | 28.1±2.2 | | 71.4±3.2 | |
| Dextromethorphan 15 mg/kg | 9 | 36.5±2.0* | 0.015 | 52.0±2.7** | 0.001 |
| CF6 20 mg/kg | 9 | 30.4±1.8 | 0.445 | 63.4±3.0 | 0.090 |
| CF6 50 mg/kg | 9 | 28.5±2.7 | 0.917 | 58.1±3.8* | 0.019 |

| | | | | | |
|---|---|---|---|---|---|
| (Compared with the solvent control group, * P<0.05, ** P<0.01) | | | | | |

### Experimental results:

1) Experimental results demonstrated that compared to the solvent control group, the dextromethorphan group exhibited significant improvements in both cough latency and cough frequency (P < 0.05), indicating statistically significant effects.
2) The CF6 (50 mg/kg) dose group showed statistically significant reduction in cough frequency versus the solvent control group.

### Experimental example 4 The pharmacodynamic test of protein CF6 (the protein in

Example 7) for expectorant effect on the phenol red excretion model in mice.
Animals: male ICR mice;
Drugs and reagents: Mucosolvan (ambroxol hydrochloride tablets), phenol red, sodium bicarbonate, protein CF6;
Instruments: centrifuge (Sigma-3K15), balance (XS105DU), Microplate reader (BIO-TEK).

### Experiment grouping:

Solvent control group;
Mucosolvan 30 mg/kg group;
Protein CF6, 20 mg/kg group, 50 mg/kg group.

### Method:

### Model preparation and administration:

Animals were fasted for 16 hours (with free access to water) prior to the experiment. A 30 mg/kg dose of Mucosolvan and different doses of protein CF6 were administered orally (administration volume: 10 mL/kg). The solvent control group were administered an equal volume of distilled water. One hour later, a 2.5% phenol red solution was injected intraperitoneally. After 30 minutes, the mice were euthanized by cervical dislocation. A segment of the trachea, extending from below the thyroid cartilage to just before the tracheal bifurcation, was excised and placed in 3 ml of 5% NaHCO₃ solution for 3 hours. Then, 1 ml of the supernatant was collected, centrifuged at 3000 rpm for 5 minutes, and the absorbance was measured and recorded at 546 nm. The excretion amount of phenol red was calculated based on its standard curve.

### Data processing:

The time points of oral administration, intraperitoneal injection of the 2.5% phenol red solution, and trachea collection were recorded separately. The absorbance of samples from each group was measured at 546 nm using a microplate reader, and the excretion amount of phenol red was calculated based on its standard curve. The mean and standard error were calculated for each group. Intergroup comparisons between the solvent control group and other groups were performed using the t-test. A value of P < 0.05 was considered statistically significant.

### Experimental results:

After administration of Mucosolvan (30 mg/kg) and different doses of protein CF6 (20 mg/kg, 50 mg/kg), a 2.5% phenol red solution was injected intraperitoneally one hour later. After 30 minutes, the mice were euthanized by cervical dislocation. A segment of the trachea, extending from below the thyroid cartilage to just before the tracheal bifurcation, was excised and placed in 3 ml of 5% NaHCO₃ solution for 3 hours. Then, 1 ml of the supernatant was collected, centrifuged at 3000 rpm for 5 minutes, and the absorbance was measured and recorded at 546 nm. The excretion amount of phenol red was calculated based on its standard curve. The results are shown in Table 5.

**Table 5. Effects of the test drug on the expectorant efficacy in the mouse phenol red excretion model (X±SEM)**

| Group | N | Phenol red excretion (µg/mL) | P |
|---|---|---|---|
| Solvent control group | 8 | 0.499±0.054 | |
| Mucosolvan 30 mg/kg | 8 | 0.866±0.052** | 0.001 |
| CF6 20 mg/kg | 8 | 0.698±0.066* | 0.035 |
| CF6 50 mg/kg | 8 | 0.605±0.075 | 0.270 |

| | | | |
|---|---|---|---|
| (Compared with the solvent control group, * P<0.05, ** P<0.01) | | | |

### Experimental results:

1) The experimental results showed that compared with the solvent control group, the amount of phenol red excretion in the Mucosolvan 30 mg/kg group was significantly increased, P<0.05, which was statistically significant.
**2)** Compared to the solvent control group, the 20 mg/kg dose group of protein CF6 showed a significant increase in phenol red excretion (P < 0.05), indicating a statistically significant difference.

## Claims

1. A keratin CF6, **characterized in that** the amino acid sequence of the keratin CF6 is:
(1) the amino acid sequence set forth in SEQ ID NO. 1 in the Sequence Listing; or
(2) an amino acid sequence formed by substitution, deletion, or addition of 1 to 45 amino acids in the amino acid sequence set forth in SEQ ID NO.1 in the sequence listing, and that basically maintains the same biological function.

2. The keratin CF6 according to claim 1, **characterized in that** the keratin CF6 can comprise a conventional modification; or be linked with a tag for detection or purification; or be homologous to keratin CF6.

3. The keratin CF6 according to claim 2, **characterized in that** the conventional modification comprises acetylation, amidation, cyclization, glycosylation, phosphorylation, alkylation, biotinylation, fluorescent group modification, polyethylene glycol (PEG) modification, immobilization modification, sulfation, oxidation, methylation, deamination, formation of disulfide bond(s), or breakage of disulfide bond(s); and the tag comprises His6, GST, EGFP, MBP, Nus, HA, IgG, FLAG, c-Myc, or Profinity eXact.

4. A nucleic acid molecule encoding the keratin CF6 according to any one of claims 1-3.

5. The nucleic acid molecule according to claim 4, **characterized in that** the nucleotide sequence of the nucleic acid molecule is:
(1) the nucleotide sequence set forth in SEQ ID NO. 2 in the Sequence Listing;
(2) a nucleotide sequence obtained by sequence optimization based on the nucleotide sequence set forth in SEQ ID NO. 2; or
(3) a nucleotide sequence complementary to the nucleotide sequence in (1) or (2) above-mentioned.

6. An expression vector, **characterized in that** the expression vector comprises the nucleic acid molecule according to any one of claims 4-5.

7. A host cell, **characterized in that** the host cell comprises the expression vector according to claim 6, or has the nucleic acid molecule according to any one of claims 4-5 integrated into its genome.

8. The host cell according to claim 7, **characterized in that** the host cell comprises bacterium, yeast, *Aspergillus*, plant cell, or insect cell.

9. The host cell according to claim 8, **characterized in that** the bacterium comprises *Escherichia coli*.

10. A method for preparing the keratin CF6 according to any one of claims 1-3, **characterized in that** it comprises the following steps:
A. synthesizing a nucleic acid molecule corresponding to the keratin CF6 according to any one of claims 1-3, ligating the nucleic acid molecule to a corresponding expression vector, transforming the expression vector into a host cell, culturing the host cell comprising the expression vector in a fermentation equipment under a certain condition, and inducing the expression of the keratin CF6 to obtain a crude protein solution comprising the keratin CF6;
B. separating, purifying, and drying the crude protein solution obtained in step A to obtain the keratin CF6.

11. The method according to claim 10, **characterized in that** in step A: the host cell is mainly selected from *Escherichia coli*; the keratin CF6 is expressed in inclusion bodies of *Escherichia coli*; and the fermentation equipment includes a shake flask or a fermentation tank.

12. The method according to claim 10, **characterized in that** in step A, after inducing the expression of the keratin CF6, removing impurities by using a detergent, dissolving the keratin CF6 in a solution to obtain a crude protein solution.

13. The method according to claim 10, **characterized in that**, in step B, the method for separating and purifying comprises ultrafiltration or microfiltration membrane technology purification method, column chromatography purification method, salting-out method, and dialysis method.

14. A pharmaceutical composition, **characterized in that** the pharmaceutical composition comprises the keratin CF6 according to any one of claims 1-3 and a pharmaceutically acceptable carrier or excipient.

15. Use of the keratin CF6 according to any one of claims 1-3, or the nucleic acid molecule according to any one of claims 4-5, or the expression vector according to claim 6, or the host cell according to any one of claims 7-9, or the pharmaceutical composition according to claim 14 in the manufacture of a medicament for antipyretic, analgesia, antitussive, expectorant, anticonvulsant, antiepileptic, hypotensive, anti-inflammatory, or antiviral.
